# FASCICULE DE BREVET EUROPEEN

(11) **EP 2 117 478 B1**
(45) Date de publication et mention de la délivrance du brevet: **22.04.2015**
(21) Numéro de dépôt: 08761849.2
(22) Date de dépôt: 07.02.2008
(51) Int. Cl.: A61F 5/01, A61F 5/30, A61F 13/10

(54) **DISPOSITIF DE SERRAGE DE L'AVANT-BRAS D'UN UTILISATEUR SOUFFRANT DE TROUBLES MUSCULAIRES DU COUDE**
VORRICHTUNG ZUM ERGREIFEN DES UNTERARMS EINES AN MUSKULÄREN PROBLEMEN IM ELLENBOGEN LEIDENDEN BENUTZERS
DEVICE FOR GRIPPING THE FOREARM OF A USER SUFFERING FROM MUSCULAR PROBLEMS IN THE ELBOW

(30) Priorité: 15.02.2007 FR 0701113
(43) Date de publication de la demande: 18.11.2009
(73) Titulaire: Gibaud, 42000 Saint Etienne (FR)
(72) Inventeur: ANGLADA, Gérard, F-42000 Saint Etienne (FR)
(74) Mandataire: Delorme, Nicolas
(86) Numéro de dépôt international: PCT/FR2008/000146
(87) Numéro de publication internationale: WO 2008/110702

(56) Documents cités:
- US-A- 5 441 058
- US-A- 5 642 739
- US-A- 5 672 150
- US-A1- 2006 118 679
- US-B1- 6 398 749

## Description

La présente invention concerne un dispositif de serrage destiné à être placé autour de l'avant-bras d'un utilisateur, pour la prévention ou le soulagement des troubles musculaires du coude.

Les troubles musculaires plus spécifiquement visés par la présente invention sont l'épicondylite et l'epitrochléite. Il s'agit de deux types de tendinite se manifestent par des douleurs au coude et sont souvent dues à une trop grande sollicitation des muscles de l'avant-bras. Ces troubles peuvent notamment apparaître suite à des mouvements répétitifs, mal exécutés ou particulièrement durs de la main et/ou du poignet, que ce soit dans le travail (menuisiers, maçons, opérateurs de marteaux-piqueurs, etc.) ou lors de la pratique d'un sport (tennis, golf, etc.) ou d'un loisir (par exemple le jardinage).

L'épicondylite apparaît lorsque le tendon des muscles extenseurs est trop sollicité ; la douleur se situe alors surtout dans la partie externe de l'avant-bras, dans la région de l'épicondyle, qui est une petite saillie osseuse de la face externe de l'humérus.

L'epitrochléite, qui est beaucoup moins fréquente, survient lorsque le tendon des muscles fléchisseurs attachés à l'épitrochlée (petite saillie osseuse de la face interne de l'humérus) est surmené ; la douleur se situe alors dans la région interne de l'avant-bras, dans la région de l'épitrochlée.

Ces deux troubles provoquent des douleurs pouvant durer quelques semaines, voire se prolonger davantage, et, s'ils sont mal soignés, peuvent dégénérer en douleurs chroniques et causer des lésions irréversibles.

S'il est préférable, au début de la crise, de mettre le coude au repos en évitant les gestes qui ont causé la lésion, en revanche, une immobilisation prolongée est à bannir car cela risque de provoquer une raideur grave de l'articulation, parfois irréversible. Puis, en phase de réadaptation, lorsque les mouvements sont repris progressivement, il peut être conseillé de porter un dispositif de serrage de l'avant-bras, qui permet de limiter les sollicitations subies par le tendon en comprimant localement le muscle. Un tel dispositif de serrage peut également être utilisé de façon préventive, en port continu lors de l'activité à risque.

On connaît déjà de tels dispositifs de serrage, qui comprennent typiquement d'une part un corps rigide mais élastiquement déformable possédant sensiblement la forme d'un U présentant un fond et une première et une deuxième branches, et d'autre part deux organes d'appui montés sur le corps chacun sensiblement à une partie extrême libre d'une branche de celui-ci.

L'utilisateur place le corps du dispositif autour de son avant-bras, dans la région adaptée, puis le serre à l'aide d'une sangle. Les organes d'appui viennent alors comprimer localement l'avant-bras, limitant ainsi l'amplitude du mouvement du tendon.

Ces dispositifs connus présentent toutefois un certain nombre d'inconvénients.

Tout d'abord, l'obtention d'un serrage satisfaisant au niveau des organes d'appui s'accompagne généralement d'un serrage trop important au niveau des autres zones de contact entre le corps et l'avant-bras. Il s'ensuit un effet de garrot, pouvant conduire à une rupture de la vascularisation, ce qui n'est bien entendu pas souhaitable.

Par ailleurs, les dispositifs connus sont souvent d'une mise en place malaisée, car le corps peut tourner autour de l'avant-bras lors du serrage par la sangle, et se trouver finalement dans une mauvaise position. De plus, il faut noter que l'épicondylite et l'epitrochléite surviennent généralement sur le coude du bras droit pour un droitier, et du bras gauche pour un gaucher, si bien que la mise en place du corps est réalisée avec la main gauche pour un droitier, et avec la main droite pour un gaucher. Ceci complique encore la mise en place du dispositif par un utilisateur seul.

En outre, les organes d'appui de certains dispositifs connus possèdent une zone effective d'appui de dimensions réduites. Ceci peut nuire à l'efficacité du dispositif, qui n'assure pas une compression satisfaisante de l'avant-bras dans la zone appropriée, mais également conduire à l'accentuation de l'effet de garrot.

On connaît par ailleurs des dispositifs de support du poignet. Ainsi, le document US 2006/118679 propose un dispositif qui comporte un corps plat noyé dans une gaine souple pourvue d'une cavité disposée sous le canal carpien. La forme classique du corps, en C, induit les inconvénients décrits ci-dessus concernant l'effet de garrot. Quant au document US 5 672 150, il décrit un dispositif qui comporte une partie supérieure et une partie inférieure placées respectivement au-dessus et en-dessous du poignet, ces parties étant enserrées par une sangle de façon à être rapprochées l'une de l'autre. Cette mise en oeuvre est relativement malaisée.

La présente invention vise à remédier aux inconvénients mentionnés ci-dessus.

A cet effet, l'invention concerne un dispositif de serrage du type mentionné ci-dessus, dans lequel au moins l'une des branches du corps comporte une première portion courbe, adjacente au fond du U, dont la concavité est dirigée vers l'intérieur du corps, et une deuxième portion courbe, prolongeant la première portion vers l'extrémité libre de la branche, dont la concavité est dirigée vers l'extérieur du corps, de sorte que ladite branche du U présente une ligne d'inflexion transversale.

Le terme « transversal » désigne la direction orthogonale à la direction générale dans laquelle s'étend la branche du U, et sensiblement contenue dans le plan moyen défini par la branche. En d'autres termes, la direction transversale correspond à la direction générale de l'avant-bras équipé d'un tel dispositif de serrage.

Avec cette ligne d'inflexion, la branche concernée du corps présente ainsi la forme de la branche d'un arc. Grâce à cette structure, lorsque le dispositif est placé sur l'avant-bras d'un utilisateur et serré par une sangle, on obtient un serrage d'une amplitude importante au niveau des organes d'appui, qui sont dirigés vers l'intérieur du corps en U et situés en regard l'un de l'autre. Ce serrage de l'avant-bras entre les organes d'appui du dispositif permet de réaliser la compression souhaitée du muscle de l'avant-bras, tandis que la force de serrage de l'avant-bras au niveau des autres zones est limitée. Ainsi, l'effet de garrot est évité. De plus, il n'est pas nécessaire pour l'utilisateur de serrer de façon importante le dispositif pour obtenir une compression localisée du muscle d'une amplitude satisfaisante, du fait de la double courbure de la ou des branches produisant un effet « bras de levier ».

Par ailleurs, du fait de cette structure du corps, la deuxième portion courbe de la branche concernée s'éloigne de l'autre branche lorsqu'on s'éloigne du fond du U. Ainsi, le U présente une ouverture évasée qui facilite l'insertion de l'avant-bras, et donc la mise en place du corps.

Avantageusement, il peut être prévu que chacune des deux branches du corps comporte une première portion courbe, adjacente au fond du U, dont la concavité est dirigée vers l'intérieur du corps, et une deuxième portion courbe, prolongeant la première portion vers l'extrémité libre de la branche, dont la concavité est dirigée vers l'extérieur du corps, de sorte que chacune des deux branches du U présente une ligne d'inflexion transversale. Les avantages précités sont ainsi encore augmentés.

La ou chaque ligne d'inflexion est par exemple située sensiblement au milieu de la branche.

Selon une réalisation possible, la largeur d'au moins une branche augmente depuis le fond du U en direction de l'extrémité libre de ladite branche. La zone de contact entre l'avant-bras et le corps dans la zone appropriée peut ainsi être augmentée.

Par exemple, au moins l'un des organes d'appui comporte une cavité intérieure dans laquelle est logée la partie extrême libre de la branche correspondante, et le dispositif de serrage comporte en outre des moyens de maintien de l'organe d'appui en position montée sur le corps.

Selon un premier mode de réalisation, ces moyens de maintien comprennent un rivet ou tout autre moyen de fixation. Selon un deuxième mode de réalisation, ces moyens de maintien comprennent des moyens d'accrochage ménagés sur ladite partie extrême libre, aptes à retenir l'organe d'appui en position montée sur le corps. Par exemple, il peut s'agir d'un crochet venant coopérer avec la face intérieure de la cavité. L'organe d'appui peut notamment être emboîté entre deux épaulements prévus sur ladite partie extrême libre.

Au moins l'une des branches peut présenter à son extrémité libre une fente agencée pour permettre le passage d'une sangle de fixation autour de l'avant-bras de l'utilisateur. En variante, la sangle peut passer dans la cavité, en étant prise en sandwich entre le corps et l'organe d'appui.

De façon avantageuse, au moins l'un des organes d'appui peut présenter, sur sa face tournée vers l'intérieur du corps, des nervures sensiblement parallèles entre elles et orientées longitudinalement.

La direction longitudinale désigne la direction générale dans laquelle s'étend la branche du U. En d'autres termes, la direction longitudinale est orthogonale aux fibres du muscle de l'avant-bras équipé d'un tel dispositif de serrage.

L'effet associé à ces nervures est de maximiser l'effort de compression et de le répartir sur une plus grande surface. Ceci vise à augmenter l'efficacité de serrage et de permettre à l'utilisateur de diminuer la douleur ressentie.

La combinaison de ces nervures avec la présence d'une ligne d'inflexion sur une ou deux branches du corps conduit à un dispositif de serrage particulièrement satisfaisant, car fournissant une compression importante localisée dans la zone appropriée (au niveau des organes d'appui) et minimisant l'effet de garrot dans les autres zones.

Il est à noter que le deuxième organe d'appui peut comporter des nervures similaires afin de jouer également le rôle d'un organe de compression. En variante, ce deuxième organe peut jouer simplement le rôle d'un contre appui. Dans ce cas, il peut être lisse, ou comporter des nervures concentriques qui visent à empêcher ou limiter la rotation du corps autour de l'avant-bras de l'utilisateur.

Au moins l'un des organes d'appui peut présenter sensiblement une forme de trapèze dont les bases sont orientées transversalement, et qui s'évase en direction de l'extrémité libre de la branche sur laquelle il est monté. On peut ainsi augmenter la surface d'appui contre l'avant-bras. Outre l'augmentation de l'efficacité de serrage, cette géométrie permet d'obtenir l'effet escompté même si l'utilisateur ne positionne pas le dispositif de façon précise sur son avant-bras.

Avantageusement, au moins l'un des organes d'appui peut être réalisé en un matériau possédant des propriétés anti-glissement, tel que le silicone ou un élastomère, afin d'éviter la rotation du corps autour de l'avant-bras de l'utilisateur lorsque le dispositif de serrage est mis en place, mais également lors des activités, éventuellement brusques, de l'utilisateur (squash, utilisation d'un marteau-piqueur, etc.). Le dispositif est de préférence porté directement sur le corps et non par-dessus les vêtements.

On décrit à présent, à titre d'exemple non limitatif, un mode de réalisation possible de l'invention, en référence aux figures annexées :
La figure 1 est une vue en perspective du dispositif de serrage selon l'invention ;
Les figures 2 et 3 sont des vues latérales, de deux côtés opposés, du dispositif de la figure 1 ;
La figure 4 est une vue en perspective d'un organe d'appui ;
La figure 5 est une vue de face du dispositif de la figure 1 ; et
La figure 6 illustre schématiquement le dispositif mis en place et serré autour de l'avant-bras d'un utilisateur.

Un dispositif 1 de serrage comprend tout d'abord un corps 2 possédant sensiblement la forme d'un U présentant un fond 3 ainsi qu'une première et une deuxième branches 4, 5. Le corps 2 est réalisé en un matériau rigide, qui permet de générer le serrage souhaité, mais élastiquement déformable, afin d'une part que les branches puissent être légèrement écartées lors de l'insertion de l'avant-bras dans le corps 2, et d'autre part que les branches puissent être courbées l'une vers l'autre lors du serrage du corps 2 par une sangle. Parmi les matériaux qui conviennent, on peut citer le polypropylène, le polyamide. L'épaisseur du corps est par exemple comprise entre 2 et 3 mm. Le corps 2 peut être fabriqué par injection.

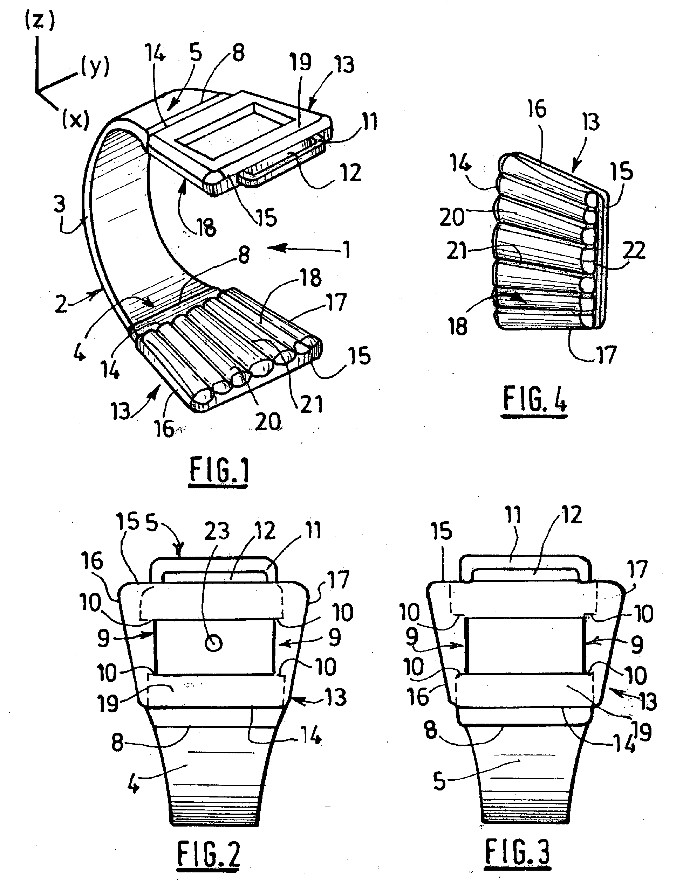

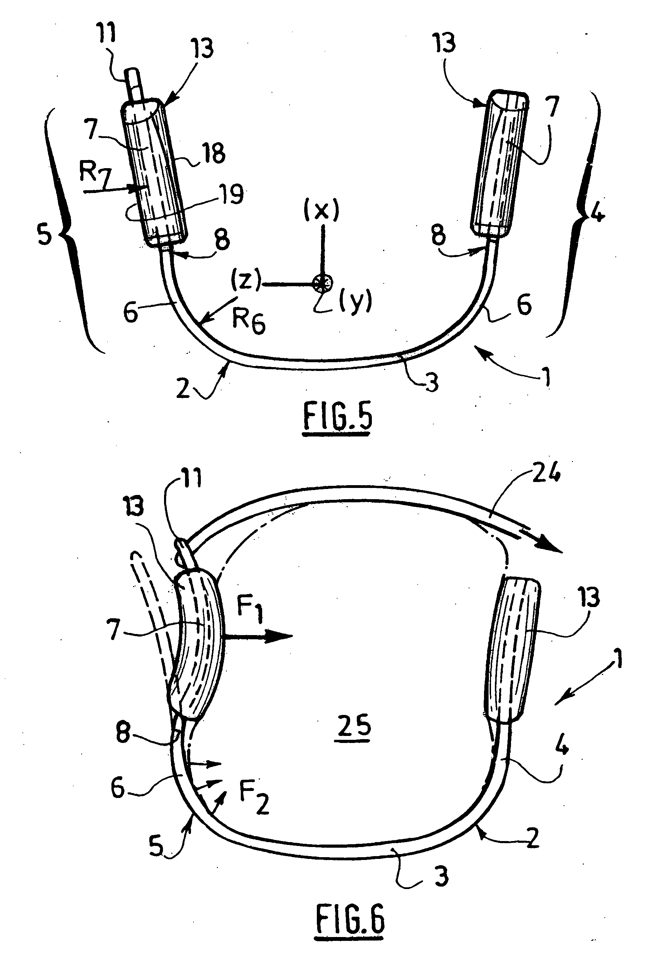

Comme illustré sur les figures 1 et 5, on définit la direction longitudinale (x) comme la direction générale dans laquelle s'étendent les branches du U et la direction transversale (y) comme la direction orthogonale à la direction longitudinale et sensiblement parallèle aux plans moyens des branches 4, 5 et du fond 3 du U. On définit également la direction (z) comme la direction orthogonale à (x) et (y).

Le fond 3 du U est arrondi ; il peut présenter une dimension selon (z) variable en fonction de la taille et de la corpulence de l'utilisateur, comprise entre 7 cm et 9 cm environ. Sur les figures est représenté un dispositif de grande taille, possédant un fond 3 comportant une partie centrale relativement plane, tandis que sur les dispositifs plus petits, le fond 3 se rapproche davantage d'un arc de cercle.

Chacune des deux branches 4, 5 comporte une première portion 6 courbe, adjacente au fond 3 du U, dont la concavité est dirigée vers l'intérieur du corps 2, et une deuxième portion 7 courbe, prolongeant la première portion 6 jusqu'à l'extrémité libre de la branche, dont la concavité est dirigée vers l'extérieur du corps 2. Le rayon de courbure R₆ de la première portion 6 est compris entre 2 et 4 cm, par exemple de l'ordre de 2,8 cm; le rayon de courbure R₇ de la deuxième portion 7 est beaucoup plus important, par exemple compris entre 9 et 11 cm.

Ainsi, entre les première et deuxième portions 6, 7 est formée une ligne d'inflexion transversale 8, et chaque branche 4, 5 présente la forme d'une branche d'arc. Dans la réalisation représentée, la ligne d'inflexion transversale 8 est située sensiblement au milieu de chaque branche 4, 5.

La largeur (selon la direction transversale) du corps 2 est de l'ordre de 3 cm au niveau du fond 3 puis augmente progressivement lorsqu'on se déplace vers les extrémités libres des branches 4, 5 jusqu'à atteindre environ 4 cm au voisinage de la ligne d'inflexion 8, et rester constante dans la deuxième portion 7 de chaque branche.

La première branche 4 (figure 2) comporte deux évidements latéraux 9 à sa partie extrême, qui forment de part et d'autre de la branche 4 (selon la direction transversale) deux épaulements 10. La deuxième branche 5 (figure 3) présente une forme similaire, et comporte de plus un prolongement 11 pourvu d'une fente 12 orientée transversalement, s'étendant sur environ 3 cm.

Le dispositif 1 de serrage comprend en outre deux organes d'appui 13, comme représenté plus spécifiquement sur la figure 4. Chaque organe d'appui 13 présente une forme générale de trapèze comportant une grande base 14, une petite base 15, et deux côtés 16, 17 symétriques par rapport à une perpendiculaire aux bases 14, 15. A titre d'exemple, la dimension de la grande base 14 peut être de l'ordre de 55 à 60 cm, et celle de la petite base 15 de l'ordre de 42 à 50 cm ; la distance entre les deux bases 14, 15 peut être de l'ordre de 4,5 à 5,5 cm.

Chaque organe d'appui 13 présente également deux faces principales, à savoir une face intérieure 18 et une face extérieure 19. La face intérieure 18 est pourvue de nervures 20 sensiblement parallèles entre elles et perpendiculaires aux bases 14, 15. Les nervures 20 prennent ici la forme de bossages allongés, dépourvus d'arêtes vives. Entre deux nervures 20 sont définis des sillons 21.

Dans chaque organe d'appui 13 est formée une cavité intérieure 22 sensiblement parallèle aux faces principales 18, 19, débouchant au moins dans la petite base 15, ainsi que sur la face extérieure 19, mais pas sur la face intérieure 18.

Les organes d'appui 13 sont réalisés en un matériau possédant des propriétés anti-glissement, tel que le silicone ou un élastomère. Ils sont montés chacun sur une partie extrême libre d'une branche 4, 5 du corps 2 du dispositif, cette partie extrême étant engagée dans la cavité 22 depuis la petite base 15, et la face intérieure 18 étant tournée vers la branche opposée, c'est-à-dire vers l'intérieur du corps 2. Les organes d'appui 13 sont emboîtés entre les deux épaulements 10.

L'organe d'appui 13 monté sur la première branche 4 est fixé à celle-ci par un rivet 23. L'organe d'appui 13 monté sur la deuxième branche 5 est traversé par celle-ci, de sorte que le prolongement 11 pourvu de la fente 12 dépasse de l'organe d'appui 13.

Une sangle 24 complète le dispositif 1 de serrage. La sangle 24 comporte par exemple deux faces principales pourvues de moyens d'accrochage de type VELCRO ®. Cette sangle 24 est insérée dans la cavité 21 de l'organe d'appui monté sur la première branche 4, à l'extérieur du corps 2, longe du côté extérieur la première branche 4, le fond 3 et la deuxième branche 5, puis passe dans la fente 12.

Lors du serrage du dispositif 1 sur l'avant-bras 25 d'un utilisateur (figure 6), les branches 4, 5 sont élastiquement déformées et rapprochées l'une de l'autre. Par exemple, la deuxième branche 5 subit sensiblement un pivotement de sa deuxième portion 7 par rapport à sa première portion 6, autour de la ligne d'inflexion 8. Ainsi, la forme en branche d'arc de la deuxième branche 5 s'accentue, la branche prenant la forme d'un S aplati.

En conséquence, on obtient un rapprochement important des deux branches 4, 5 au niveau des organes d'appui 13, et donc un serrage important dans la zone correspondante de l'avant-bras, symbolisé par la flèche F1. En revanche, dans les autres zones périphériques de l'avant-bras, en particulier au niveau des premières portions 6 des branches 4, 5 qui se sont peu rapprochées l'une de l'autre, la force de serrage F2 appliquée à l'avant-bras est moindre, et l'effet de garrot est évité. La pression exercée sur l'avant-bras est donc localisée au niveau des organes d'appui 13.

La forme évasée de l'ouverture du corps puis le rapprochement, lors du serrage, des deux branches permet également d'éviter le pincement de la peau lors de la mise en place du dispositif 1.

La surface d'appui (face intérieure 18) étant importante, il n'est pas nécessaire pour l'utilisateur de réaliser un positionnement très précis du corps 2, contrairement aux dispositifs de l'art antérieur

Généralement l'un des organes d'appui joue le rôle de moyen de compression du muscle, et est placé du côté approprié de l'avant-bras selon le trouble musculaire à prévenir / soulager. Les nervures 20 de cet organe d'appui 13 sont orientées perpendiculairement aux fibres du muscle, ce qui maximise l'effort de compression. L'autre organe d'appui joue le rôle de contre appui et, de préférence, de moyen empêchant le glissement. Il n'est donc pas nécessairement pourvu de nervures perpendiculaires aux fibres du muscle.

Le dispositif, existant en plusieurs tailles, est adapté à une mise en place sur un bras gauche ou droit. Eventuellement, le corps peut comporter un repère visuel sur sa face extérieure, tel qu'un logo, pour indiquer à l'utilisateur le sens correct de mise en place. En effet, on peut prévoir un dispositif de serrage dont les deux branches du corps ont sensiblement la même longueur et présentent toutes deux une double courbure. Dans ce cas, le dispositif de serrage possède sensiblement un plan de symétrie, qui est parallèle à la direction générale dans laquelle s'étendent les branches du U et à la ligne d'inflexion. Le logo facilite donc la mise en place du dispositif malgré cette symétrie.

Il va de soi que l'invention n'est pas limitée au mode de réalisation décrit ci-dessus à titre d'exemple mais qu'elle en embrasse au contraire toutes les variantes de réalisation.

## Revendications

1. Dispositif de serrage destiné à être placé autour de l'avant-bras d'un utilisateur, pour la prévention ou le soulagement des troubles musculaires du coude, comprenant d'une part un corps (2) rigide mais élastiquement déformable possédant sensiblement la forme d'un U présentant un fond (3) et une première et une deuxième branches (4, 5), et d'autre part deux organes d'appui (13) montés sur le corps (2) chacun sensiblement à une partie extrême libre d'une branche (4, 5) de celui-ci, **caractérisé en ce qu'**au moins l'une des branches du corps (2) comporte une première portion courbe (6), adjacente au fond (3) du U, dont la concavité est dirigée vers l'intérieur du corps (2), et une deuxième portion courbe (7), prolongeant la première portion (6) vers l'extrémité libre de la branche, dont la concavité est dirigée vers l'extérieur du corps (2), de sorte que ladite branche du U présente une ligne d'inflexion (8) transversale.

2. Dispositif de serrage selon la revendication 1, **caractérisé en ce que** chacune des deux branches (4, 5) du corps (2) comporte une première portion courbe (6), adjacente au fond (3) du U, dont la concavité est dirigée vers l'intérieur du corps (2), et une deuxième portion courbe (7), prolongeant la première portion (6) vers l'extrémité libre de la branche, dont la concavité est dirigée vers l'extérieur du corps (2), de sorte que chacune des deux branches (4, 5) du U présente une ligne d'inflexion (8) transversale.

3. Dispositif de serrage selon la revendication 1 ou 2, **caractérisé en ce que** la ou chaque ligne d'inflexion (8) est située sensiblement au milieu de la branche (4, 5).

4. Dispositif de serrage selon l'une des revendications 1 à 3, **caractérisé en ce que** la largeur d'au moins une branche (4, 5) augmente depuis le fond (3) du U en direction de l'extrémité libre de ladite branche.

5. Dispositif de serrage selon l'une des revendications 1 à 4, **caractérisé en ce qu'**au moins l'un des organes d'appui (13) comporte une cavité intérieure (22) dans laquelle est logée la partie extrême libre de la branche (4, 5) correspondante, et **en ce que** le dispositif de serrage (1) comporte en outre des moyens de maintien (9, 10) de l'organe d'appui en position montée sur le corps (2).

6. Dispositif de serrage selon l'une des revendications 1 à 5, **caractérisé en ce qu'**au moins l'une des branches (4, 5) présente à son extrémité libre une fente (12) agencée pour permettre le passage d'une sangle (24) de fixation autour de l'avant-bras de l'utilisateur.

7. Dispositif de serrage selon l'une des revendications 1 à 6, **caractérisé en ce qu'**au moins l'un des organes d'appui (13) présente, sur sa face (18) tournée vers l'intérieur du corps (2), des nervures (20) sensiblement parallèles entre elles et orientées longitudinalement.

8. Dispositif de serrage selon l'une des revendications 1 à 7, **caractérisé en ce qu'**au moins l'un des organes d'appui (13) présente sensiblement une forme de trapèze dont les bases (14, 15) sont orientées transversalement, et qui s'évase en direction de l'extrémité libre de la branche (4, 5) sur laquelle il est monté.

9. Dispositif de serrage selon l'une des revendications 1 à 8, **caractérisé en ce qu'**au moins l'un des organes d'appui (13) est réalisé en un matériau possédant des propriétés anti-glissement, tel que le silicone ou un élastomère.

## Patentansprüche

1. Vorrichtung zum Ergreifen, die dazu bestimmt ist, um den Unterarm eines Benutzers platziert zu werden, um muskulären Störungen des Ellenbogens vorzubeugen oder sie zu lindern, die einerseits einen starren aber elastisch verformbaren Körper (2) umfasst, der im Wesentlichen die Form eines U besitzt, das einen Grund (3) und einen ersten und einen zweiten Schenkel (4, 5) aufweist, und andererseits zwei Stützorgane (13), die auf dem Körper (2) montiert sind, jeder im Wesentlichen an einem freien Endteil seiner Schenkel (4, 5), **dadurch gekennzeichnet, dass** mindestens einer der Schenkel des Körpers (2) einen ersten gebogenen Abschnitt (6) umfasst, der mit dem Grund (3) des U, dessen Konkavität zu dem Inneren des Körpers (2) gerichtet ist, benachbart ist, und einen zweiten gebogenen Abschnitt (7), der den ersten Abschnitt (6) zu dem freien Ende des Schenkels verlängert, dessen Konkavität zu der Außenseite des Körpers (2) derart gerichtet ist, dass der Schenkel des U eine transversale Biegungslinie (8) aufweist.

2. Vorrichtung zum Ergreifen nach Anspruch 1, **dadurch gekennzeichnet, dass** jeder der zwei Schenkel (4, 5) des Körpers (2) einen ersten gebogenen Abschnitt (6) umfasst, der mit dem Grund (3) des U, dessen Konkavität zu dem Inneren des Körpers (2) gerichtet ist, benachbart ist, und einen zweiten gebogenen Abschnitt (7), der den ersten Abschnitt (6) zu dem freien Ende des Schenkels verlängert, dessen Konkavität zu der Außenseite des Körpers (2) derart gerichtet ist, dass jeder der zwei Schenkel (4, 5) des U eine transversale Biegungslinie (8) aufweist.

3. Vorrichtung zu Ergreifen nach Anspruch 1 oder 2, **dadurch gekennzeichnet, dass** die oder jede Biegungslinie (8) im Wesentlichen in der Mitte des Schenkels (4, 5) liegt.

4. Vorrichtung zum Ergreifen nach einem der Ansprüche 1 bis 3, **dadurch gekennzeichnet, dass** die Breite mindestens eines Schenkels (4, 5) von dem Grund (3) des U in Richtung des freien Endes des Schenkels zunimmt.

5. Vorrichtung zum Ergreifen nach einem der Ansprüche 1 bis 4, **dadurch gekennzeichnet, dass** mindestens eines der Stützorgane (13) einen inneren Hohlraum (22) umfasst, in dem der freie Endteil des entsprechenden Schenkels (4, 5) aufgenommen ist, und dass die Vorrichtung zum Ergreifen (1) außerdem Haltemittel (9, 10) des Stützorgans in auf dem Körper (2) montierter Position umfasst.

6. Vorrichtung zum Ergreifen nach einem der Ansprüche 1 bis 5, **dadurch gekennzeichnet, dass** mindestens einer der Schenkel (4, 5) an seinem freien Ende einen Schlitz (12) aufweist, der eingerichtet ist, um die Passage eines Befestigungsgurts (24) um den Unterarm des Benutzers zu erlauben.

7. Vorrichtung zum Ergreifen nach einem der Ansprüche 1 bis 6, **dadurch gekennzeichnet, dass** mindestens eines der Stützorgane (13) auf seiner Seite (18), die zu dem Inneren des Körpers (2) gerichtet ist, Rippen (20) aufweist, die im Wesentlichen zueinander parallel und längs ausgerichtet sind.

8. Vorrichtung zum Ergreifen nach einem der Ansprüche 1 bis 7, **dadurch gekennzeichnet, dass** mindestens eines der Stützorgane (13) im Wesentlichen eine Trapezform aufweist, deren Basen (14, 15) quer ausgerichtet sind, und das sich in Richtung des freien Endes des Schenkels (4, 5) auf das es montiert ist, erweitert.

9. Vorrichtung zum Ergreifen nach einem der Ansprüche 1 bis 8, **dadurch gekennzeichnet, dass** mindestens eines der Stützorgane (13) aus einem Werkstoff hergestellt ist, der Rutschsicherheitseigenschaften besitzt, wie zum Beispiel Silikon oder Elastomer.

## Claims

1. A gripping device intended to be placed around the forearm of a user, for preventing or alleviating muscular problems in the elbow, comprising a stiff but elastically deformable body (2) substantially having the shape of a U, having a bottom (3) and first and second limbs (4, 5) on the one hand, and two supporting members (13) each substantially mounted on the body (2) to a free utmost portion of a limb (4, 5) of the latter on the other hand, **characterized in that** at least one of the limbs of the body (2) includes a first curved portion (6) adjacent to the bottom (3) of the U, the concavity of which is oriented towards the inside of the body (2), and a second curved portion (7) extending the first portion (6) towards the free end of the limb, the concavity of which is oriented towards the outside of the body (2), so that said limb of the U has a transverse inflection line (8).

2. The gripping device according to claim 1, **characterized in that** each of the two limbs (4, 5) of the body (2) includes a first curved portion (6), adjacent to the bottom (3) of the U, the concavity of which is oriented towards the inside of the body (2) and a second curved portion (7) extending the first portion (6) towards the free end of the limb, the concavity of which is oriented towards the outside of the body (2), so that each of the two limbs (4, 5) of the U has a transverse inflection line (8).

3. The gripping device according to claim 1 or 2, **characterized in that** said or each inflection line (8) is substantially located in the middle of the limb (4, 5).

4. The gripping device according to any of claims 1 to 3, **characterized in that** the width of at least one limb (4, 5) increases from the bottom (3) of the U towards the free end of said limb.

5. The gripping device according to any of claims 1 to 4, **characterized in that** at least one of the supporting members (13) includes an inner cavity (22) in which the free utmost portion of the corresponding limb (4, 5) is housed, and **in that** the gripping device (1) further includes means (9, 10) for holding the supporting member in position mounted on the body (2).

6. The gripping device according to any of claims 1 to 5, **characterized in that** at least one of the limbs (4, 5) has at its free end a slot (12) laid out in order to provide the passage for an attachment strap (24) around the forearm of the user.

7. The gripping device according to any of claims 1 to 6, **characterized in that** at least one of the supporting members (13) has, on its face (18) turned towards the inside of the body (2), ribs (20) substantially parallel to each other and oriented longitudinally.

8. The gripping device according to any of claims 1 to 7, **characterized in that** at least one of the supporting members (13) substantially has a trapezium shape, the bases (14, 15) of which are oriented transversely, and which flares towards the free end of the limb (4, 5) on which it is mounted.

9. The gripping device according to any of claims 1 to 8, **characterized in that** at least one of the supporting members (13) is made in a material having anti-sliding properties, such as silicone or an elastomer.
